(19) Europäisches Patentamt
European Patent Office
Office européen des brevets

(11) Veröffentlichungsnummer: **0 022 906**
**B1**

(12) **EUROPÄISCHE PATENTSCHRIFT**

(45) Veröffentlichungstag der Patentschrift: **08.08.84**

(21) Anmeldenummer: **80102672.5**

(22) Anmeldetag: **14.05.80**

(51) Int. Cl.³: **C 07 D 231/12**, C 07 D 231/14 // C07C109/14, C07D295/06, C07D231/06, C07C87/29

(54) **Verfahren zur Herstellung von Pyrazol-Derivaten.**

(30) Priorität: **21.05.79 DE 2990941**

(43) Veröffentlichungstag der Anmeldung: **28.01.81 Patentblatt 81/4**

(45) Bekanntmachung des Hinweises auf die Patenterteilung: **08.08.84 Patentblatt 84/32**

(84) Benannte Vertragsstaaten: **AT BE CH DE FR GB IT LI LU NL SE**

(56) Entgegenhaltungen:
**DE - A - 2 536 003**
**DE - C - 748 539**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Biere, Helmut, Dr., Zeltinger Strasse 15, D-1000 Berlin 28 (DE)**

**EP 0 022 906 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft den in dem Patentanspruch gekennzeichneten Gegenstand.

Pyrazol-Derivate der allgemeinen Formel I gemäß Patentanspruch sind aus der deutschen Patentanmeldung P 2 633 992 bekannt, ihre Herstellung nach den bekannten Verfahren ist aber sehr aufwendig und die erzielten Ausbeuten sind oft unbefriedigend.

Demgegenüber gelingt es mit Hilfe des erfindungsgemäßen Verfahrens die Pyrazol-Derivate der allgemeinen Formel I mittels einer einfachen Synthese in guten Ausbeuten darzustellen.

So erhält man beispielsweise die 4-(4-Chlorphenyl)-1-(4-fluorphenyl)-3-pyrazolessigsäure nach dem vorbekannten Verfahren in 29% der Theorie, nach dem erfindungsgemäßen Verfahren hingegen in 65% der Theorie bezogen auf das eingesetzte 4-Chloranilin.

Das erfindungsgemäße Verfahren wird unter an sich bekannten Bedingungen durchgeführt (Org. Reactions, 3, 1946, 83—107; Angew. Chem., 70, 1958, 351—367 und Synthesis 1975, 358—375).

Das erfindungsgemäße Verfahren wird in Gegenwart primärer oder sekundärer Amine durchgeführt. Geeignete Amine sind beispielsweise Methylamin, Äthylamin, Dimethylamin, Diäthylamin, Dibutylamin, Anilin, $\alpha$-, $\beta$- oder $\gamma$-Picolin, Piperidin oder insbesondere Morpholin und Pyrrolidin.

Die nachfolgenden Ausführungsbeispiele dienen zur Erläuterung des erfindungsgemäßen Verfahrens.

Beispiel 1

a) 28,9 g 4-Fluoranilin werden mit 260 ml 15%iger Salzsäure und 260 g Eis versetzt, auf —5° C gekühlt und mit 19,7 g Natriumnitrit in 50 ml Wasser diazotiert. Unter ständigem Rühren und Kühlen wird eine Lösung aus 38,4 g 3-Chlor-2,4-pentandion, 260 ml Äthanol, 260 g Eiswasser und 350 g Natriumacetat-Hydrat zugetropft. Die Mischung wird noch 3 Stunden bei 0° C gerührt, dann mit Wasser verdünnt, wobei die Substanz auskristallisiert. Der Kristall-Rückstand wird aus Äthanol umkristallisiert; man erhält 50 g 1-Chlor-1-(4-fluorphenylhydrazono)-2-propanon mit Schmelzpunkt 148° C.

b) Eine Lösung von 19,6 g 4-Brombenzylcyanid in 100 ml Toluol wird tropfenweise bei —10° C unter Stickstoff mit 108 ml einer 20%igen Lösung von Diisobutylaluminiumhydrid (Dibah) in Toluol versetzt und anschließend noch 15 Minuten gerührt. Nach vorsichtiger Zugabe von 35 ml Äthanol, wird mit 250 ml Wasser ca. 20 ml konzentrierter Schwefelsäure (bis pH 2) versetzt und 15 Minuten bei 0°C gerührt. Danach wird die Toluolphase abgetrennt, die wäßrige Phase mit Toluol nachextrahiert, die vereinigte Toluollösung mit Wasser gewaschen und über Magnesiumsulfat getrocknet. Nach Filtration wird die Toluollösung des 4-Chlorphenylacetaldehyds mit 87 g Morpholin versetzt und langsam im Vakuum auf das halbe Volumen konzentriert, dann über Nacht stehengelassen. Nach Einengen der Lösung wird der Rückstand mit Cyclohexan verrührt, wobei Kristallisation erfolgt. Das Kristallisat wird anschließend aus Diisopropyläther umkristallisiert; man erhält 12 g 4-(4-Bromstyryl)-morpholin mit Schmelzpunkt 142° C.

c) Eine Lösung von 7,2 g 4-(4-Bromstyryl)-morpholin in 30 ml Chloroform wird nacheinander mit 2,7 g Triäthylamin und einer Lösung von 5,3 g 1-Chlor-1-(4-fluorphenylhydrazono)-2-propanon in 30 ml Chloroform versetzt und über Nacht bei Raumtemperatur gerührt (alternativ kann 4 Stunden unter Rückfluß gekocht werden). Nach dem Waschen mit 2 N Salzsäure. Natriumhydrogencarbonat-Lösung und Wasser wird die Chloroformphase mit Magnesiumsulfat getrocknet und eingeengt. Der aus Hexan (Cyclohexan) kristallisierte Rückstand (10,5 g) kann entweder weiterverarbeitet oder noch einmal aus Äthanol umkristallisiert werden; man erhält 3-Acetyl-4-(4-bromphenyl)-1-(4-fluorphenyl)-5-morpholino-4,5-dihydropyrazol mit Schmelzpunkt 148° C.

d) Eine Lösung von 10 g des obengenannten Dihydropyrazols in 110 ml Dioxan wird mit 30 ml 2 N Salzsäure 60—90 Minuten unter Rückfluß gekocht. Danach wird im Vakuum eingeengt, der Rückstand in Äthylacetat aufgenommen, mit Wasser gewaschen, mit Magnesiumsulfat getrocknet und im Vakuum eingeengt. Der Rückstand wird aus Methanol kristallisiert und ergibt 7,5 g 3-Acetyl-4-(4-bromphenyl)-1-(4-fluorphenyl)-pyrazol mit Schmelzpunkt 185° C.

e) Eine Mischung von 7,2 g des 3-Acetylpyrazol-Derivates und 0,84 g Schwefel in 9 ml Morpholin wird 3—4 Stunden auf 135° C erhitzt, danach in 150 ml Eiswasser gegossen und gut verrührt. Der abgesaugte Niederschlag wird aus Äthylacetat und Dioxan kristallisiert; man erhält 7,8 g 4-[4-(4-Bromphenyl)-1-(4-fluorphenyl)-3-pyrazolyl-thioacetyl]-morpholin mit Schmelzpunkt 211° C.

f) Eine Mischung von 7,4 g gemäß Beispiel 1d hergestellten Thioacetylmorpholin-Derivats in 20 ml Dimethylsulfoxid wird mit 6,4 g Natriumhydroxid in 30 ml Wasser versetzt und 5 Stunden unter Rückfluß erhitzt. Nach Eingießen in Eiswasser kristallisiert das Natriumsalz aus; es wird abgesaugt, mit Methylenchlorid gewaschen und in 2 N Salzsäure suspendiert. Man erhält 5 g 4-(4-Bromphenyl)-1-(4-fluorphenyl)-3-pyrazol-essigsäure mit Schmelzpunkt 175° C.

## Beispiel 2

Analog Beispiel 1 wird 4-(4-Chlorphenyl)-1-(4-fluorphenyl)-3-pyrazol-essigsäure mit Schmelzpunkt 148° C (Äthanol/Wasser) erhalten.
Die verwendeten Zwischenprodukte sind:

4-(4-Chlorstyryl)-morpholin, Schmelzpunkt 102° C (Diisopropyläther) (dargestellt analog Beispiel 1b aus 4-Chlorbenzylcyanid)
3-Acetyl-4-(4-chlorphenyl)-1-(4-fluorphenyl)-5-morpholino-4,5-dihydropyrazol, Schmelzpunkt 137° C (Äthanol)
3-Acetyl-4-(4-chlorphenyl)-1-(4-fluorphenyl)-pyrazol, Schmelzpunkt 181° C (Isopropylalkohol)
4-[4-(4-Chlorphenyl)-1-(4-fluorphenyl)-3-pyrazolyl-thioacetyl]-morpholin, Schmelzpunkt 213° C (Dioxan)
4-(4-Chlorphenyl)-1-(4-fluorphenyl)-3-pyrazol-essigsäure, Natriumsalz, Schmelzpunkt 299° C (Wasser).

Die Darstellung von 3-Acetyl-4-(4-chlorphenyl)-1-(4-fluorphenyl)-pyrazol kann auch analog Beispiel 1c aus α-Dimethylamino-4-chlorstyrol und 1-Chlor-1-(4-fluorphenylhydrazono)-propan-2-on erfolgen.
Das Ausgangsmaterial α-Dimethylamino-4-chlorstyrol kann auf folgende Weise gewonnen werden:

Eine Lösung von 8 g 4-Chlorbenzylchlorid in 50 ml Äther wird tropfenweise zu 1,5 g Magnesiumspäne (unter Argon) gegeben und 30 Minuten nachgerührt. Anschließend wird die Lösung auf —15° C abgekühlt und tropfenweise mit 11 g reinem Dimethylformamid und 30 ml Tetrahydrofuran versetzt. Das Reaktionsgemisch wird 3 Stunden bei —15° C gerührt, dann im Vakuum vorsichtig eingeengt und mit 50 ml 2 N Ammoniumchloridlösung sowie Äthylacetat versetzt. Der organische Rückstand wird aus Methanol umkristallisiert; man erhält 5 g α-Dimethylamino-4-chlorstyrol mit Schmelzpunkt 77° C.

## Beispiel 3

Analog Beispiel 1 wird 1,4-Bis(4-chlorphenyl)-3-pyrazol-1-essigsäure mit Schmelzpunkt 184° C (Toluol) erhalten.
Die verwendeten Zwischenprodukte sind:

1-Chlor-1-(4-chlorphenylhydrazono)-2-propanon, Schmelzpunkt 175° C (Äthanol) (dargestellt analog Beispiel 1a aus 4-Chloranilin).
3-Acetyl-1,4-bis(4-chlorphenyl)-pyrazol, Schmelzpunkt 190° C (Dioxan)
4-[1,4-Bis(4-chlorphenyl)-3-pyrazolyl-thioacetyl]-morpholin, Schmelzpunkt 204° C (Dioxan)

## Beispiel 4

Analog Beispiel 1 wird 4-(4-Chlorphenyl)-1-(4-methyl-thiophenyl)-3-pyrazol-essigsäure mit Schmelzpunkt 169° C (Methanol) erhalten.
Die verwendeten Zwischenprodukte sind:

1-Chlor-1-(4-methylthiophenylhydrazono)-2-propanon, Schmelzpunkt 110° C (Tetrachlormethan); (dargestellt analog Beispiel 1a aus 4-Methylthioanilin).
3-Acetyl-4-(4-chlorphenyl)-1-(4-methylthiophenyl)-pyrazol, Schmelzpunkt 85° C (Methanol)
4-[4-(4-Chlorphenyl)-1-(4-methylthiophenyl)-3-pyrazolyl-thioacetyl]-morpholin, Schmelzpunkt 248° C (Äthylacetat)

## Beispiel 5

Eine Lösung von 1,8 g 4-(4-Chlorphenyl)-1-(4-methylthiophenyl)-3-pyrazol-essigsäure (dargestellt nach Beispiel 4) in einem Gemisch von 20 ml Chloroform und 20 ml Essigsäure wird mit 1 ml Wasserstoffperoxid (30%) versetzt und bei Raumtemperatur eine Stunde gerührt. Danach wird mit Wasser verdünnt, wobei die Substanz auskristallisiert. Die kristalline Substanz wird abgesaugt, mit heißem Wasser gewaschen und aus Methanol umkristallisiert; man erhält 1,4 g 4-(4-Chlorphenyl)-1-(4-methylsulfinylphenyl)-3-pyrazol-essigsäure mit Schmelzpunkt 193° C.

Beispiel 6

Analog Beispiel 1 wird 4-(4-Chlorphenyl)-1-phenyl-3-pyrazol-essigsäure mit Schmelzpunkt 169° C (Toluol) erhalten.

Die verwendeten Zwischenprodukte sind:

1-Chlor-1-phenylhydrazono-2-propanon, Schmelzpunkt 136° C (Äthanol)
3-Acetyl-4-(4-chlorphenyl)-1-phenyl-pyrazol, Schmelzpunkt 101° C (Methanol)
4-[4-(4-Chlorphenyl)-1-phenyl-3-pyrazolyl-thioacetyl]-morpholin, Schmelzpunkt 205° C (Dioxan).

Beispiel 7

Eine Lösung von 2 g 4-(4-Chlorphenyl)-1-(4-fluorphenyl)-3-pyrazol-essigsäure (dargestellt nach Beispiel 2) in 30 ml Dimethylformamid wird mit einer Lösung von 600 mg Kupfer(II)-acetat in 35 ml Dimethylformamid versetzt und 8 Stunden bei Raumtemperatur gerührt. Nach Abdestillieren des Lösungsmittels im Vakuum wird der ölige Rückstand mit Aceton gelöst und von Rückständen abfiltriert. Nach Verdampfen des Acetons wird der Salzrückstand aus Methanol kristallisiert. Man erhält 1,7 g 4-(4-Chlorphenyl)-1-(4-fluorphenyl)-3-pyrazolessigsäure-Kupfer(II)-salz-Monohydrat vom Schmelzpunkt 171° C.

Beispiel 8

Analog Beispiel 1 wird die 1,4-Bis(4-fluorphenyl)-3-pyrazolessigsäure (Schmelzpunkt 116° C aus Tetrachlormethan) hergestellt.

Beispiel 9

Analog Beispiel 1 wird die 4-(2,4-Dichlorphenyl)-1-(4-fluorphenyl)-3-pyrazolessigsäure (Schmelzpunkt 133° C aus Toluol) hergestellt.

Beispiel 10

Analog Beispiel 1 wird die 1-(2-Fluorphenyl)-4-(4-nitrophenyl-3-pyrazolessigsäure (Schmelzpunkt 208° C aus Essigester) hergestellt.

Beispiel 11

Analog Beispiel 1 wird die 4-(4-Chlorphenyl)-1-(2-fluorphenyl)-3-pyrazolessigsäure (Schmelzpunkt 187° C aus Essigester) hergestellt.

Beispiel 12

Analog Beispiel 1 wird die 1-(4-Fluorphenyl)-4-(4-nitrophenyl)-3-pyrazolessigsäure (Schmelzpunkt 254° C aus Acetonitril) hergestellt.

Beispiel 13

Analog Beispiel 1 wird die 4-(3,4-Dichlorphenyl)-1-(4-fluorphenyl)-3-pyrazolessigsäure (Schmelzpunkt 178° C aus Toluol) hergestellt.

**Patentanspruch**

Verfahren zur Herstellung von Pyrazol-Derivaten der allgemeinen Formel I

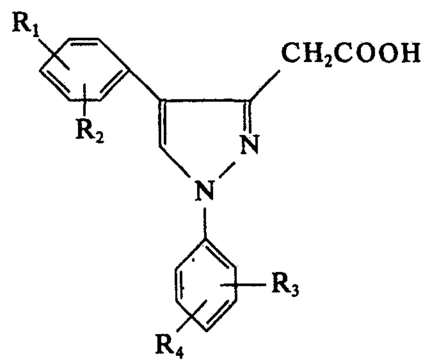

(I)

worin $R_1$, $R_2$, $R_3$ und $R_4$ ortho-, meta- oder para-ständige Substituenten sind und ein Wasserstoffatom, ein Halogenatom, eine Alkylgruppe, eine Alkoxygruppe, eine Trifluormethylgruppe, eine Nitrogruppe, eine Aminogruppe, eine Alkylthio oder eine Alkylsulfonylgruppe darstellen, mit der Maßgabe, daß mindestens einer der Substituenten $R_1$ bis $R_4$ von Wasserstoff verschieden ist,
dadurch gekennzeichnet,
daß man eine Verbindung der allgemeinen Formel II

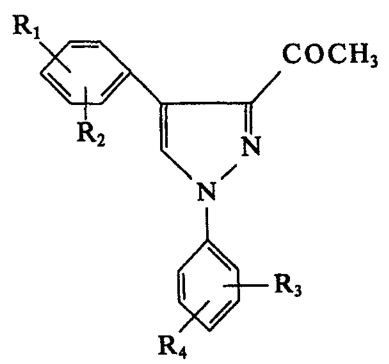

(II)

worin $R_1$, $R_2$, $R_3$ und $R_4$ die obengenannte Bedeutung besitzen mit Schwefel und einem Amin der allgemeinen Formel III

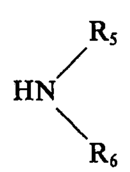

(III)

worin
$R_5$ und $R_6$ gemeinsam eine Tetramethylengruppe, eine Pentamethylengruppe oder eine Äthoxyäthylengruppe darstellen
oder worin
$R_5$ ein Wasserstoffatom oder eine Alkylgruppe mit 1 bis 4 Kohlenstoffatomen und
$R_6$ eine Alkylgruppe mit 1 bis 4 Kohlenstoffatomen, eine Phenylgruppe oder eine Pyridylgruppe bedeuten, umsetzt,
und die so erhaltene Verbindung der allgemeinen Formel IV

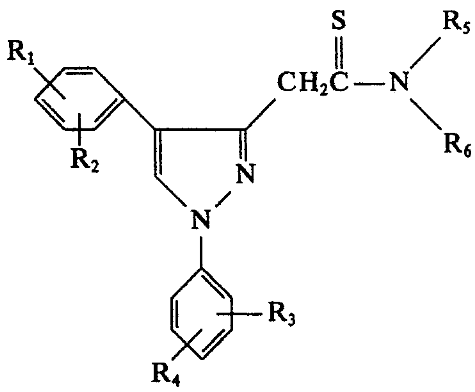

(IV)

worin $R_1$ bis $R_6$ die obengenannte Bedeutung besitzen, mittels Säuren oder Basen hydrolysiert.

**Claim**

Process for the manufacture of pyrazole derivatives of the general formula I

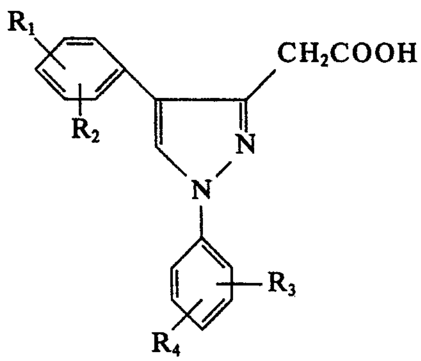

(I)

in which $R_1$, $R_2$, $R_3$ and $R_4$ are substituents in the ortho-, meta- or para-positions and represent a hydrogen atom, a halogen atom, an alkyl group, an alkoxy group, a trifluoromethyl group, a nitro group, an amino group, an alkylthio group or an alkylsulphonyl group, with the proviso that at least one of the substituents $R_1$ to $R_4$ is other than hydrogen,
characterized in that
a compound of the general formula II

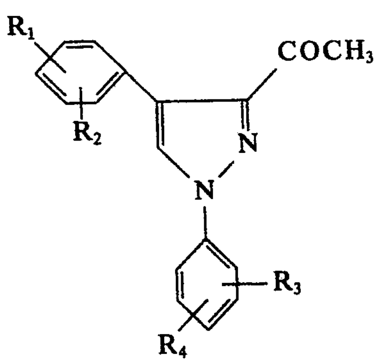

(II)

in which $R_1$, $R_2$, $R_3$ and $R_4$ have the above-mentioned meanings, is reacted with sulphur and an amine of the general formula III

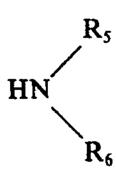

(III)

in which
$R_5$ and $R_6$
together represent a tetramethylene group, a pentamethylene group or an ethoxyethylene group, or in which
$R_5$ represents a hydrogen atom or an alkyl group having from 1 to 4 carbon atoms and
$R_6$ represents an alkyl group having from 1 to 4 carbon atoms, a phenyl group or a pyridyl group,
and the resulting compound of the general formula IV

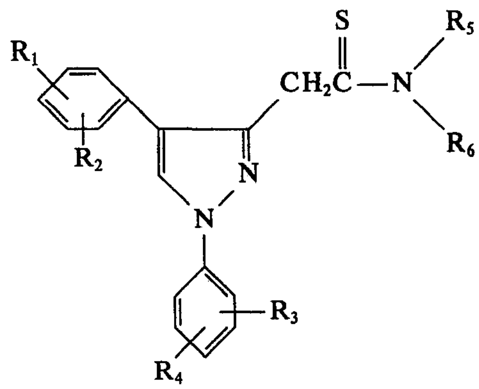

(IV)

in which $R_1$ to $R_6$ have the above-mentioned meanings, is hydrolysed using acids or bases.

**Revendication**

Procédé de préparation de dérivés du pyrazole répondant à la formule générale (I):

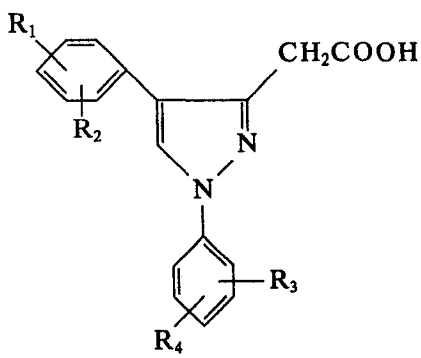

(I)

dans laquelle $R_1$, $R_2$, $R_3$ et $R_4$ peuvent se trouver aux positions ortho, méta ou para et représentent chacun un atome d'hydrogène ou d'halogène ou un radical alkyle, alcoxy, trifluorométhyle, nitro, amino, alkylthio ou alkylsulfonyle, avec la condition qu'au moins l'un des symboles $R_1$ à $R_4$ ne représente pas l'hydrogène, procédé caractérisé en ce qu'on fait réagir un composé répondant à la formule générale (II):

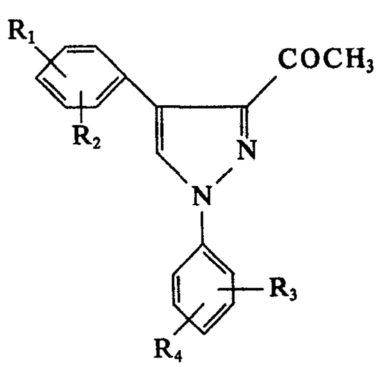

(II)

dans laquelle $R_1$, $R_2$, $R_3$ et $R_4$ ont les significations qui viennent d'être données, avec le soufre et avec une amine répondant à la formule générale (III):

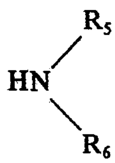

(III)

dans laquelle:

$R_5$ et $R_6$ forment ensemble un radical tétraméthylène, pentaméthylène ou éthoxy-éthylène

ou dans laquelle:

$R_5$ représente un atome d'hydrogène ou un radical alkyle contenant de 1 à 4 atomes de carbone et

$R_6$ représente un radical alkyle contenant de 1 à 4 atomes de carbone, un radical phényle ou un radical pyridyle,

et on hydrolyse le composé ainsi obtenu, qui répond à la formule générale (IV):

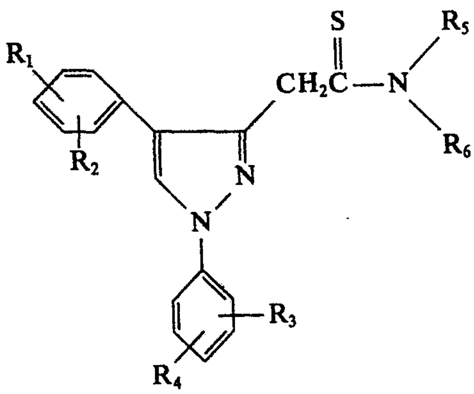

(IV)

dans laquelle $R_1$ à $R_6$ ont les signification précédemment données, au moyen d'acides ou de bases.